# EUROPEAN PATENT SPECIFICATION

(11) **EP 1 722 759 B1**
(45) Date of publication and mention of the grant of the patent: **09.03.2011**
(21) Application number: 05717745.3
(22) Date of filing: 18.02.2005
(51) Int. Cl.: A61K 9/00

(54) **COMPOSITION CONTAINING CHITOSAN AND A POLYOL-PHOSPHATE OR A SUGAR-PHOSPHATE**
ZUSAMMENSETZUNG ENTHALTEND CHITOSAN SOWIE EIN ZUCKERPHOSPHAT ODER EIN POLYOLPHOSPHAT
COMPOSITION CONTENANT DU CHITOSANE ET UN PHOSPHATE DE SUCRE OU UN PHOSPHATE DE POLYOL

(30) Priority: 21.02.2004 GB 0403938
(43) Date of publication of application: 22.11.2006
(73) Proprietor: Archimedes Development Limited, Nottingham, Nottinghamshire NG7 2TN (GB)
(72) Inventor: DYER, Ann, Margaret, Archimedes Development Ltd., University Blvd, Nottingham NG7 2TN (GB); PASTOR, Patricia, Archimedes Development Ltd., University Blvd, Nottingham NG7 2TN (GB); ILLUM, Lisbeth, Nottingham NG7 1BA (GB)
(74) Representative: Crowhurst, Charlotte Waveney
(86) International application number: PCT/GB2005/000592
(87) International publication number: WO 2005/079749

(56) References cited:
- WO-A-01/36000
- WO-A-02/40072
- WO-A-96/03142
- US-A- 5 422 116
- US-A1- 2003 158 302
- HOFFMAN A S ET AL: "RELEASE OF CYTOKINE RECEPTORS FROM PHYSICAL HYDROGELS OF PLURONIC POLYETHERS GRAFTED TO CHITOSAN BACKBONES" PROCEEDINGS OF THE INTERNATIONAL SYMPOSIUM ON CONTROLLED RELEASE BIOACTIVE MATERIALS, vol. 24, 1997, pages 126-127, XP001029276 ISSN: 1022-0178

## Description

This invention relates to pharmaceutical compositions that provide for the uptake of therapeutic agents across mucosal surfaces.

Polar drugs, including low molecular weight drugs, high molecular weight peptides, proteins and polysaccharides, are not typically effectively absorbed across mucosal membranes, such as the gastrointestinal tract, the oral mucosal, the eye, the vagina, the nasal cavity or the rectum.

The use of "absorption enhancers" such as non-ionic surfactants, cyclodextrins, phospholipids and bile salts to improve the absorption of polar molecules across mucosal membranes has previously been described. (For a review see Davis et al (eds.), Delivery Systems for Peptide Drugs, Plenum Press, New York, 1987; and Lee (ed.), Peptide and Protein Delivery, Marcel Dekker Inc., New York, 1991).

Chitosan is a cationic biopolymer comprising glucosamine and N-acetyl glucosamine that has bioadhesive properties and has been shown to improve the systemic bioavailability of certain drug compounds across mucosal surfaces such as the nasal cavity (see Illum, Drug Discovery Today, 7, 1184-1189, 2002*).*

Injectable neutral solutions of chitosan which form biodegradable gels in-situ are described in the literature (A. Chenite et al., Biomaterials 21: 2155-2161 (2000); E. Ruel-Gariepy et al., Int. J. Pharm. 203: 89-98 (2000); A. Chenite et al., Carbohydrate Polymers 46: 39-47 (2001)) and the patent literature WO01/36000, WO99/07416 and US-B-6344488). In-situ gel formation is facilitated by the addition of an anionic polyol-phosphate salt to the chitosan solution, this is reported to result in the neutralisation of the positively charged chitosan and the solution pH. When injected in vivo the liquid formulations are converted into gel implants in-situ and may be used to deliver biologically active molecules and as encapsulating matrices for tissue engineering applications (A. Chenite et al., Carbohydrate Polymers 46: 39-47 (2001)).

US 9422116 discloses a sustained release liquid aqueous ophtalmic composition comprising chitosan.

It is an object of the present invention to provide a composition suitable for the delivery of therapeutic agents across a mucosal surface. In particular, the present invention is concerned with the provision of a solution that gels at physiological temperature, thereby prolonging the residence time of the therapeutic agent on the mucosal surface.

More particularly, in view of the known advantages of the use of chitosan in compositions for the transportation of drugs across mucosal membranes, such as the nasal cavity, it would be particularly advantageous to provide chitosan compositions that initially have a low viscosity but form a gel at physiological temperature such that a gel is formed shortly after application to a mucosal surface. This has not previously been possible because compositions comprising chitosan at relatively high concentration tend to have a high viscosity and may, therefore, be difficult to deliver, for example using a nasal spray device. Additionally, the onset of gelation using prior art compositions can be prolonged.

The present invention, therefore, provides a composition according to claim 1.

The compositions of the invention may be in any suitable form. As the person of ordinary skill in the art will appreciate, suitable forms will depend on the intended method of administration. The compositions of the invention are in the form of an aqueous solution or an aqueous composition in which the therapeutic agent is suspended.

By the term "chitosan" we include all derivatives of chitin, or poly-N-acetyl-D-glucosamine, including all polyglucosamines and oligomers of glucosamine materials of different molecular weights, in which the greater proportion of the N-acetyl groups have been removed through hydrolysis (deacetylation). In accordance with the present invention, the degree of deacetylation, which represents the proportion of N-acetyl groups that have been removed through deacetylation, should preferably be greater than 40 %, for example from 40 to 97%, more preferably from 50 to 98%, more preferably from 60 to 95% and most preferably from 70 to 90%.

The chitosan, chitosan derivative or salt used in the present invention should preferably have a molecular weight of 4000 Daltons (Da) or greater, more preferably from 10,000 to 2,000,000 Da, more preferably from 25,000 to 1,000,000 Da and most preferably from 50,000 to 300,000 Da.

Salts of chitosan are suitable for use in the present invention. Salts with various organic and inorganic acids are suitable. Such suitable salts include, but are not limited to the nitrate, phosphate, glutamate, lactate, sulphate, citrate, hydrochloride and acetate salts. Preferably, the glutamate or hydrochloride salt is used.

Chitosan derivatives and their salts are also suitable for use in this invention. Suitable chitosan derivatives are derivatives formed by bonding acyl and/or alkyl groups with the hydroxyl groups, but not the amino groups of chitosan. Examples include O-alkyl ethers of chitosan, O-acyl esters of chitosan, trimethyl chitosan and similar derivatives. Modified chitosans, such as those conjugated to polyethylene glycol may be used in the present invention. Conjugates of chitosan and polyethylene glycol are described in WO99/01498.

Chitosans suitable for use in the present invention may be obtained from various sources, including Primex, Haugesund, Norway; NovaMatrix, Drammen, Norway; Seigagaku America Inc., MD, USA; Meron (India) Pvt, Ltd., India; Vanson Ltd, VA, USA; and AMS Biotechnology Ltd., UK. Suitable derivatives include those that are disclosed in Roberts, Chitin Chemistry, MacMillan Press Ltd., London (1992).

Particularly preferred chitosan compounds that may be mentioned include chitosan glutamate (available as Protasan UPG213 from NovaMatrix, Drammen, Norway) and other low and medium viscosity chitosan compounds (for example UPG113, UPCL213 and UPCL113 grades also available from NovaMatrix, Drammen, Norway).

As will be appreciated, the amount of chitosan, a salt or derivative thereof or salt of a derivative thereof present in the compositions of the present invention will, at least to some extent, depend on factors such as the other components present, their concentration and the intended mode of administration. If the composition of the invention is in the form of an aqueous solution or suspension, the chitosan, salt or derivative thereof or salt of a derivative thereof is preferably present in an amount of from 0.25 to 3.0 %w/v, more preferably from 0.35 to 2.5 %w/v, for example from 0.35 to 2.0 %w/v, from 0.5 to 2.5 %w/v, from 0.75 to 2.0 %w/v or from 0.4 to 1 %w/v and most preferably from 0.45 to 1.5 %w/v expressed as chitosan base.

Preferably, the chitosan used in the present invention has a positive charge in solution.

The chitosan, salt or derivative thereof or salt of a derivative thereof used in the present invention is preferably water soluble. By the term "water soluble", we mean that the chitosan, salt or derivative thereof or salt of a derivative thereof has a solubility of at least 1 mg/ml and preferably at least 10 mg/ml in water at ambient temperature.

The compositions of the present invention comprise a polyol-phosphate or sugar-phosphate. These terms and the alternative terms mono-phosphate dibasic salts of a polyol or a sugar will be well understood by those skilled in the art and include, but are not limited to, all salts or derivatives of glycerol-, sorbitol-, xylitol-, mannitol-, fructose-, glucose-, galactose-, ribose-, xylose-, trehalose-, sucrose-phosphate or mixtures thereof. Preferably, a salt or derivative of glycerol, sorbitol, fructose or glucose is used. Most preferably, a salt or derivative of glycerol is used.

Preferably, the polyol-phosphate is β-glycerophosphate or glycerol-2-phosphate and most preferably β-glycerophosphate disodium is used in the present invention.

As will be appreciated, the amount of polyol-phosphate or sugar-phosphate salt present in the compositions of the present invention will, at least to some extent, depend on factors such as the other components present, their concentration and the intended mode of administration. If the final composition of the invention is in the form of an aqueous solution or suspension, it preferably contains from 0.25 to 3.0 % w/v, more preferably from 0.5 to 2.5 % w/v and most preferably from 0.75 to 2.0 % w/v of the polyol-phosphate or sugar-phosphate salt. It is particularly preferred that the compositions of the invention contain β-glycerophosphate disodium in these amounts.

The compositions of the invention comprise a plasticizer. By the term "plasticizer" we mean a material that is able to interact on a molecular level with the chitosan, salt or derivative thereof or salt of a derivative thereof and thus alter certain physical and mechanical properties of the chitosan, salt or derivative thereof or salt of a derivative thereof by enhancing the mobility of the polymer chains. Without wishing to be bound by theory, it is thought that the plasticizer has the effect of reducing the temperature at which gelation of the compositions of the invention occurs by modifying the electrostatic and hydrophobic interactions and hydrogen bonding between chitosan chains, which are the main forces involved in gel formation. It will be appreciated that the nature and amount of the plasticizer used in the compositions of the invention can be selected so that gelation of the composition occurs within a specified temperature range.

The plasticizer used in the present invention is triethyl citrate.

As will be appreciated, the amount of the plasticizer included in the compositions of the present invention will, at least to some extent, depend on the nature and amounts of the other components of the composition and the intended mode of administration. Preferably, the final compositions of the invention comprise from 0.05 to 5.0 %w/v of the plasticizer, more preferably from 0.1 to 2.0 %w/v and most preferably from 0.2 to 1.0 %w/v. For example, a composition of the invention may contain triethyl citrate in an amount within these ranges.

The compositions of the invention comprise a therapeutic agent. The term "therapeutic agent" encompasses any substance that may be used to prevent or treat conditions or diseases of the animal body, including the human body and includes drugs, peptides, proteins, polysaccharides, genes (DNA) or gene constructs, vaccines or components thereof (for example isolated antigens or parts thereof) and monoclonal antibodies.

Preferably the therapeutic agent is a polar molecule. By the term "polar molecule" we mean molecules with a partition coefficient between water and octanol at pH 7.4 of less than 50, preferably less than 10.

The therapeutic agents that may be used in the present invention include, but are not limited to, insulin, PTH (parathyroid hormone), PTH analogues, PTHrP (human parathyroid hormone peptide), calcitonins (for example porcine, human, salmon, chicken or eel) and synthetic modifications thereof, enkephalins, LHRH (luteinising hormone releasing hormone) and analogues (nafarelin, buserelin, leuprolide, goserelin), glucagon, TRH (thyrotropine releasing hormone), vasopressin, desmopressin, growth hormone, heparins, GHRH (growth hormone releasing hormone) CCK (cholecystokinin), THF (thymic humoral factor), CGRP (calcitonin gene related peptide), atrial natriuretic peptide, nifedipine, metoclopramide, ergotamine, pizotizin, pentamidine and vaccines (for example, AIDS vaccines, measles vaccines, rhinovirus Type 13 and respiratory syncytial virus vaccines, influenza vaccines, pertussis vaccines, meningococcal vaccines, tetanus vaccines, diphtheria vaccines, cholera vaccines and DNA vaccines (such as one containing a plasmid DNA coding for a suitable antigen)).

Further therapeutic agents include, but are not limited to, antibiotics and antimicrobial agents, such as tetracycline hydrochloride, leucomycin, penicillin, penicillin derivatives, erythromycin, sulphathiazole and nitrofurazone; antimigrane compounds, such as naratriptan, sumatriptan, zolmitriptan, rizatriptan, eletriptan, frovatriptan, alnitidan, avitriptan, almotriptan or other 5-HT1 agonists; vasoconstrictors (such as phenylephedrine hydrochloride, tetrahydrozoline hydrochloride, naphazoline nitrate, oxymetazoline hydrochloride and tramazoline hydrochloride, cardiotonics (such as digitalis and digoxin), vasodilators (such as nitroglycerin and papaverine hydrochloride), bone metabolism controlling agents (such as vitamin D and active vitamin D3), sex hormones, hypotensive, anti-tumour agents, steroidal anti-inflammatory agents (such as hydrocortisone, prednisone, fluticasone, prednisolone, triamcinolone, triamcinolone acetonide, dexamethasone, betamethasone, beclomethasone and beclomethasone dipropionate), non-steriodal anti-inflammatory drugs (such as acetaminophen, aspirin, aminopyrine, phenylbutazone, mefenamic acid, ibuprofen, diclofenac sodium, indomethacin, colchicines and probenecid), enzymatic anti-inflammatory agents (such as chymotrypsin and bromelain seratiopeptidase), antihistaminic agents (such as diphenhydramine hydrochloride, chlorpheniramine maleate and clemastine), anti-tussive expectorants (such as codeine phosphate and isoproterenol hydrochloride), analgesics such as opioids (such as diamorphine, hydromorphone, buprenorphine, fentanyl, oxycodone, codeine, morphine and its polar metabolites, such as morphine-6-glucuronides and morphine-3-sulphate), combinations of opioids and other analgesic agents (such as non-steriodal anti-inflammatory drugs), antiemetics (such as metoclopramide, ondansetron, chlorpromazine), drugs for treatment of epilepsy (such as clonazepam), drugs for treatment of sleeping disorders (such as melatonin), drugs for treatment of asthma (such as salbutamol), drugs for treatment of erectile dysfunction (such as apomorphine, sildenafil and alprostadil).

Preferred therapeutic agents for use in the present invention include calcitonin, sumatriptan, sildenafil, apomorphine, alprostadil, buprenorphine, fentanyl, morphine and hydromorphone.

Two or more of the therapeutic agents listed above may be used in combination in the present invention. The therapeutic agents listed above may also be used with therapeutic agents other than those listed above. If the compositions of the invention contain more than one therapeutic agent, it is not necessary for each drug to have improved therapeutic effect as a result of its inclusion in a composition of the invention.

As the person of ordinary skill in the art will appreciate, the amount of the therapeutic agent incorporated into the compositions of the invention will depend on a number of factors such as the proposed dosing regimen, the route of administration and the potency of the therapeutic agent. The amount of the therapeutic agent incorporated into the compositions of the invention will typically be in the range 0.001 mg to 1000 mg.

The therapeutic agent(s) are preferably present in the compositions of the invention in solution or as a suspension.

The compositions of the invention may contain one or more excipients that reduce the viscosity of the composition prior to gel formation. Suitable viscosity reducing excipients include, but are not limited to, organic acids such as ascorbic acid, fumaric acid, malic acid and tartaric acid; triethyl citrate; polysorbates such as polysorbate 80; polyethylene glycols; and propylene glycol. The person of ordinary skill in the art would be able to readily determine suitable quantities of such excipients depending on factors such as the identity of the active ingredient(s) and the viscosity of the solution. It should also be noted that certain polyol-phosphate or sugar-phosphate salts such as β-glycerophosphate also reduce the viscosity of the compositions of the invention.

It is particularly advantageous that the compositions of the invention contain an excipient that reduces the viscosity of the composition if the composition in the absence of such an excipient has viscosity above about 150 cP. The amount of excipient that reduces the viscosity of the composition can be selected so that the composition has a viscosity suitable for the intended mode of administration while retaining desirable gelling properties.

In a preferred aspect of the invention, the compositions contain ascorbic acid. Preferably, the compositions of the present invention comprise from 0.001 to 0.5 %w/v of ascorbic acid, more preferably from 0.005 to 0.25 %w/v and most preferably from 0.01 to 0.2 %w/v as measured in relation to the total concentration of ascorbic acid in the composition.

The compositions of the present invention may also contain other pharmaceutically acceptable ingredients well known in the art. Such ingredients include, but are not limited to, antioxidants or antioxidant synergists or mixtures thereof (for example, ascorbic acid, ascorbyl palmitate, fumaric acid, malic acid, tartaric acid, sodium ascorbate or sodium metabisulphite or their synergists for example disodium edentate), chelating agents (such as edetic acid or one of its salts), preservatives (such as potassium sorbate, parabens, phenylethyl alcohol or benzalkonium chloride), flavours, sweeteners, thickening, adhesive or gelling agents, including, but not limited to, celluloses such as hydroxypropyl methylcellulose, methylcellulose, hydroxypropyl cellulose, sodium carboxyl cellulose and microcrystalline cellulose, poloxamers, polyethylene glycols, carbomers or polyethylene oxide.

It will be appreciated that some ingredients may have more than one function when used in the compositions of the invention. For example organic acids, such as ascorbic acid, fumaric acid, malic acid, tartaric acid, may act as both a viscosity reducer and an antioxidant if an appropriate concentration of the acid is used.

Preferably the compositions of the invention contain a preservative and/or are sterile. If preservatives are omitted from the compositions, microorganisms may be removed using any suitable method known in the art, for example by making the compositions aseptically or by terminally sterilising them.

If the compositions of the invention contain a preservative, any suitable known preservative may be used. Suitable preservatives include, but are not limited to, benzalkonium chloride, benzethonium chloride, methyl hydroxybenzoate, phenylethyl alcohol, propyl hydroxybenzoate and sodium benzoate. Preferably the preservative is benzalkonium chloride. The person of ordinary skill in the art will be readily able to optimise the amount of preservative for a particular composition.

Preferably the compositions of the invention are non-pyrogenic.

As will be appreciated, the preferred viscosity of the compositions of the invention prior to gelling will, at least to some extent, depend on the intended mode of administration. The skilled person will appreciate what viscosities are suitable for particular modes of administration. The compositions of the invention preferably have a viscosity of 150 mPa·s (cP) or less, more preferably 100 mPa·s (cP) or less and most preferably 50 mPa·s (cP) or less, when measured at 25°C following manufacture and using a calibrated Brookfield DV-III Programmable Rheometer fitted with a CP40 cone and plate and a sample volume of 500 µl, a rotation speed of 0.1 rpm and an equilibration time of 3 minutes. Compositions having viscosities within these ranges are particularly suitable for intranasal administration in the form of a spray but they may also be used in other forms of administration such as vaginal, rectal, oral mucosal, ophthalmic or ocular administration. As the skilled person will appreciate, the nasal route of administration is one of the most demanding in terms of suitable viscosity ranges for the compositions. It is possible that compositions having a viscosity of greater than 150 mPa·s (cP) may be used in one or more of the other forms of administration.

The compositions of the invention are typically solutions or suspensions at ambient temperature (for example below 30 °C, such as at about 20 to 25 °C). The compositions of the invention form gels when subjected to higher temperature, for example temperatures of 30 °C or greater. Preferably, the compositions of the invention form gels at temperatures of from 30 to 40 °C. More preferably, the compositions of the invention gel at a physiological temperature such as 35 to 37°C (35, 36 or 37°C), for example at the temperature within the nose (about 35 °C).

Ideally, the compositions of the invention form a gel shortly after being subjected to a temperature suitable for inducing gelling. Preferably the gel is formed in 30 minutes or less, more preferably in 15 minutes or less, more preferably in 10 minutes or less and most preferably in 5 minutes or less.

By the term "gel" we mean a transparent or translucent semi-solid or solid preparation comprising organic macromolecules distributed uniformly throughout a liquid in such a way that no apparent boundaries exist between the dispersed macromolecules and the liquid.

The preferred compositions of the invention form a gel at physiological temperature (35 to 37 °C) in 15 minutes or less on contact with a mucosal membrane following administration, more preferably in 10 minutes or less following administration and most preferably in 5 minutes or less following administration.

The compositions of the invention can be used for delivery of a therapeutic agent across a mucosal membrane into the systemic circulation.

The compositions of the invention may be administered via the nasal or ocular routes.

The preferred route of administration is the nasal route. The compositions of the invention may be administered to the nasal cavity in any suitable form. For example, when the compositions are in the form of a solution or a suspension, they may be administered in the form of drops or a spray.

A preferred method of administering the compositions of the invention that are in the form of solutions or suspensions is using a spray device. Spray devices can be single ("unit") dose or multiple dose systems, for example comprising a bottle, pump and actuator, and are available from various commercial sources, including Pfeiffer (Germany), Valois (France), Calmar (Germany), Ursatech (Germany), Bespak (UK) and Becton-Dickinson (USA). Electrostatic spray devices, such as described in US 5,655,517, are also suitable for the intranasal administration of the solutions of the invention.

For a spray device, the typical volume of liquid that is dispensed in a single spray actuation is from 0.01 to 0.14 ml, for example from 0.05 to 0.14 ml, such as 0.1 ml. It is a practical proposition to administer up to about 0.2 ml into each nostril (i.e. two x 0.1 ml sprays) to provide a therapeutic dose of drug, although the most acceptable dosing regimen would be one spray into one or both nostrils.

If a composition of the invention, in the form of a solution or suspensions, is to be administered to the ophthalmic surfaces, commercially available spray devices fitted with the appropriate actuator may be used, such as those available from Valois (France). Spray delivery devices suitable for preservative free systems in the form of multidose non-venting pumps are available for mucosal drug delivery to surfaces such as those of the nose, the ear and the buccal route. Such devices can be obtained from the sources listed above, for example from Valois (France) or Ursatech (Germany).

The present invention also provides a drug delivery device, such as a drug delivery device suitable for delivery of a composition via the nasal, or ocular routes or a dose cartridge for use with such a device loaded with a composition as defined above.

The present invention also provides a process for the preparation of the composition described above, which process comprises mixing a solution comprising chitosan or a salt or derivative thereof or a salt of a derivative thereof with a solution comprising a polyol-phosphate or sugar-phosphate salt. Other components of the compositions may be present in either the chitosan containing solution or the polyol-phosphate or sugar-phosphate salt containing solution or may be introduced into the mixture separately.

The present invention also provides the use of the combination of chitosan or a salt or derivative thereof or the salt of a derivative thereof, a polyol-phosphate or sugar-phosphate salt and a plasticizer in the manufacture of a medicament for use in the transport of a therapeutic agent across a mucosal surface in an animal (such as a mammal, for example a human) and the use of this combination in the manufacture of a medicament for nasal ophthalmic or ocular delivery. Medicaments produced in this way are for systemic action.

The present invention also provides the use of a composition as described above in the manufacture of a medicament for use in the transport of a therapeutic agent across a mucosal surfaces in an animal (such as a mammal, for example a human) and in the manufacture of a medicament for nasal, ophthalmic or ocular delivery. Medicaments produced in this way are for systemic action.

The compositions of the present invention may be used in the administration of a therapeutic agent for transport of that therapeutic agent across a mucosal surface in an animal (such as a mammal, for example a human), for example in nasal, ophthalmic or ocular delivery of a therapeutic agent to an animal. The compositions of the invention are used in the delivery of therapeutic agent is intended for systemic action.

The compositions of the invention may be used to treat/prevent diseases/conditions in mammalian patients depending upon the therapeutic agent(s) which is/are employed. For the above, non-exhaustive, lists of drugs, diseases/conditions which may be mentioned including those against which the therapeutic agent(s) in question are known to be effective, include those specifically listed for the drugs in question in Martindale "The Extra Pharmacopoeia", 33rd Edition, Royal Pharmaceutical Society (2002).

It is an advantage of the present invention that the formation of the gel prolongs the residence time between the chitosan and the active moiety and the mucosal surface. Without wishing to be bound by theory, it is thought that the compositions of the invention can enhance the delivery of the therapeutic agent into the mucosal tissue to provide, for example, increased absorption of systemically-acting drugs, peptides and proteins into the blood circulation, improved presentation of vaccine antigens to the underlying lymphoid tissue, and enhanced transfection by DNA of the cells of the mucosal lining.

The chitosan or a salt or derivative thereof or a salt of a derivative thereof in the compositions of the present invention preferably has a positive charge. The polyol-phosphate or sugar-phosphate neutralises the charge on the chitosan or a salt or derivative thereof or a salt of a derivative thereof. Thus, the ratio of the polyol-phosphate or sugar-phosphate to the chitosan or a salt or derivative thereof or a salt of a derivative thereof should be such that not all of the positive charge on the chitosan or a salt or derivative thereof or a salt of a derivative thereof is neutralised. The ratio of the polyol-phosphate or sugar-phosphate to the chitosan or a salt or derivative thereof or a salt of a derivative thereof will depend on a number of factors such as the molecular weight and degree of deacetylation of the chitosan or a salt or derivative thereof or a salt of a derivative thereof, the identity and molecular weight of the polyol-phosphate or sugar-phosphate and the percentage neutralisation of the chitosan or a salt or derivative thereof or a salt of a derivative thereof. On the basis of this information, the person of ordinary skill in the art would be able to calculate a suitable ratio of the polyol-phosphate or sugar-phosphate to the chitosan or a salt or derivative thereof or a salt of a derivative thereof.

Preferably less than 100 %, more preferably less than 90 % and most preferably less than 80 % of the positive charge on the chitosan or a salt or derivative thereof or a salt of a derivative thereof is neutralised in the composition of the invention.

The following are examples of the number of moles of certain polyolphosphates required to neutralise certain chitosan materials. Chitosan glutamate having a degree of deacetylation of 83% requires 3.4 moles of PO₄²⁻ (glycerophosphate) to neutralise each mole of chitosan (NH₃⁺). Chitosan glutamate having a degree of deacetylation of 87% requires 3.3 moles of PO₄²⁻ (glycerophosphate) to neutralise each mole of chitosan (NH₃⁺). Chitosan base having a degree of deacetylation of 92.6% requires 1.6 moles of PO₄²⁻ (glycerophosphate) to neutralise each mole of chitosan (NH₃⁺). Chitosan hydrochloride having a degree of deacetylation of 87% requires 2.1 moles of PO₄²⁻ (glycerophosphate) to neutralise each mole of chitosan (NH₃⁺). As described in Ruel-Gariepy et al., Int. J. Pharm., 203 (2000), 89-98, Chitosan glutamate having a degree of deacetylation of 84% requires 2.6 moles of PO₄²⁻ (glycerophosphate) to neutralise each mole of chitosan (NH₃⁺). To ensure that the chitosan or a salt or derivative thereof or a salt of a derivative thereof retains a positive charge, the amount of the polyol-phosphate or sugar-phosphate used in the compositions of the invention should be less than the amount that provides the number of moles of PO₄²⁻ required to neutralise the chitosan or a salt or derivative thereof or a salt of a derivative thereof.

In the Figures:

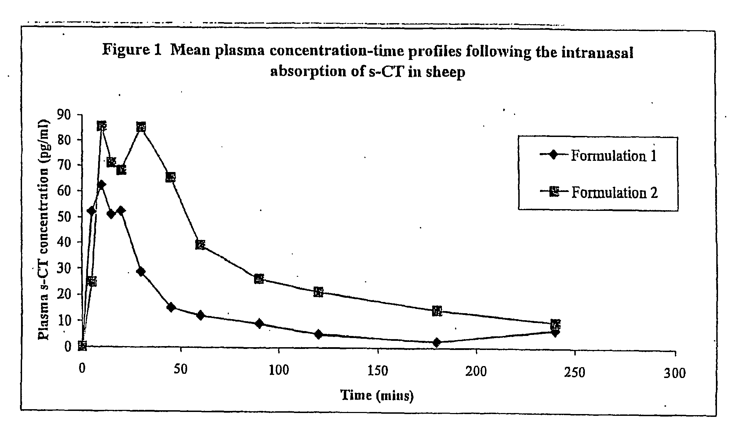

Figure 1 shows mean plasma concentration-time profiles following the intranasal absorption of s-CT (salmon calcitonin) in sheep obtained in Example 18.

The invention is illustrated by the following non-limiting Examples.

Examples i to iv are Comparative Examples and illustrate formulations which either failed to gel at physiological temperature and/or in which the solution viscosity is considered too high, thus rendering the product unsuitable for delivery using commercially available spray devices.

### Comparative Example i : Chitosan solution containing chitosan glutamate 18.8 mg/ml

Approximately 8 ml ultrapure water (Elga) was dispensed into a glass beaker and 188 mg chitosan glutamate (UP G213, FMC BioPolymer AS, Drammen, Norway) was slowly added with stirring. The contents were stirred until dissolution occurred. The solution was transferred to a 10 ml volumetric flask, the washings transferred and the contents made up to volume with ultrapure water and mixed.

| Composition | | mg/ml |
|---|---|---|
| Chitosan glutamate | | 18.8 |
| Ultrapure water | **to** | 1 ml |
| Solution pH | | 5.0 |
| Onset of gelation (37 °C) | | No gel formation |
| Viscosity of solution | | 307.5 mPa·s (cP) |
| *(control solution)* | | |

The onset of gelation of a 100 µl dose was determined qualitatively at physiological temperature using a pre-warmed glass microscope slide placed in an oven and a spatula to probe the sample. The viscosity (n=3) of the solution was determined following manufacture at 25 °C using a calibrated Brookfield DV-III Programmable Rheometer fitted with a CP40 cone and plate and a sample volume of 500 µl, a rotation speed of 0.1 rpm and an equilibration time of 3 minutes. Comparative Example i failed to gel at physiological temperature using the technique described.

### Comparative Example ii: Chitosan solution containing chitosan glutamate 18.8 mg/ml and triethyl citrate 6.6 mg/ml

### Part A Chitosan solution

152 mg triethyl citrate NF (Morflex Inc, North Carolina, USA) was weighed into a glass beaker and approximately 16 ml ultrapure water added. 434 mg chitosan glutamate was slowly added and the contents stirred until dissolution occurred. The solution was transferred to a 20 ml volumetric flask, the washings transferred and the contents made up to volume with ultrapure water and mixed.

### Part B Chitosan solution

4.6 ml of cold chitosan solution Part A (chilled on ice) was dispensed into a glass beaker using a Gilson Microman pipette and 0.709 ml ultrapure water was added. Stirring was stirring continued for a further 5 minutes. The onset of gelation and the solution viscosity were determined as detailed previously.

| Composition | | mg/ml |
|---|---|---|
| Chitosan glutamate | | 18.8 |
| Triethyl citrate | | 6.6 |
| Ultrapure water | **to** | 1 ml |
| | | |
| Solution pH | | 4.8 |
| Onset of gelation (37 °C) | | No gel formation |
| Viscosity of solution | | 288.1 mPa·s (cP) |

Comparative Example ii failed to gel at physiological temperature using the technique described in Comparative Example i.

### Comparative Example iii: Chitosan solution containing chitosan glutamate 18.8 mg/ml and β-glycerophosphate disodium 14.1 mg/ml

### Part A Chitosan solution

Approximately 8 ml ultrapure water was dispensed into a glass beaker and 217 mg chitosan glutamate was slowly added with stirring. The contents were stirred until dissolution occurred. The solution was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate (Sigma Aldrich, UK) solution 150 mg/ml (chilled on ice; prepared by dissolving 3 g of β-glycerophosphate disodium in ultrapure water in a 20 ml volumetric flask and making up to volume with ultrapure water) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Ultrapure water | **to** | 1 ml |
| Solution pH | | 6.4 |
| Onset of gelation (3 5 °C) | | 15-20 minutes |
| Viscosity of solution | | 178.8 mPa·s (cP) |

Comparative Example iii exhibited an acceptable onset of gelation at physiological temperature using the technique described previously, although the high viscosity made the product unsuitable for dosing in vivo.

### Comparative Examples iv: Chitosan solution containing chitosan glutamate 18.8 mg/ml, β-glycerophosphate disodium 14.1 mg/ml and triethyl citrate 5 mg/ml

### Part A Chitosan solution

57.5 mg triethyl citrate was weighed into a glass beaker and approximately 7.5 ml ultrapure water added. 217 mg chitosan glutamate was slowly added with stirring. The contents were stirred until dissolution occurred. The solution was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate solution 150 mg/ml (chilled on ice) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Triethyl citrate | | 5 |
| Ultrapure water | **to** | 1 ml |
| | | |
| Solution pH | | 6.4 |
| Onset of gelation (35°C) | | 10-15 minutes |
| Viscosity of solution | | 173.7 mPa·s (cP) |

Comparative Example iv exhibited an acceptable rapid onset of gelation at physiological temperature using the technique described previously, although the high viscosity made the product unsuitable for dosing in vivo.

### Reference Example 1: Self-gelling chitosan solution containing chitosan glutamate 18.8 mg/ml, β-glycerophosphate disodium 14.1 mg/ml, triethyl citrate 5mg/ml and ascorbic acid 0.1 mg/ml

### Part A Chitosan solution

57.5 mg triethyl citrate was weighed into a glass beaker and approximately 7.5 ml ultrapure water added. 0.23 ml ascorbic acid solution 5 mg/ml (prepared by dissolving and making up to volume, 100 mg ascorbic acid (Sigma Aldrich, UK) in ultrapure water in a 20 ml volumetric flask) was added to the beaker with stirring. 217 mg chitosan glutamate was slowly added and the contents stirred until dissolution occurred. The solution was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate solution 150 mg/ml (chilled on ice) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Triethyl citrate | | 5 |
| Ascorbic acid | | 0.1 |
| Ultrapure water | **to** | 1 ml |
| | | |
| Solution pH | | 6.4 |
| Onset of gelation (35°C) | | 0-5 minutes |
| Viscosity of solution | | 55.2 mPa·s (cP) |

Reference Example 1, containing a chitosan glutamate concentration of 18.8 mg/ml, exhibited a rapid onset of gelation at 35 °C using the technique described previously and a significantly lower viscosity than a control solution of similar polymer concentration. It is thought that the reduction in viscosity was due to the presence of ascorbic acid in the composition of Example 1.

### Reference Example 2: Self-gelling chitosan solution containing chitosan glutamate 18.8 mg/ml, β-glycerophosphate disodium 14.1 mg/ml, triethyl citrate 5 mg/ml and ascorbic acid 0.5 mg/ml

### Part A Chitosan solution

57.5 mg triethyl citrate was weighed into a glass beaker and approximately 6.5 ml ultrapure water added. 1.15 ml ascorbic acid solution 5 mg/ml was added to the beaker with stirring. 217 mg chitosan glutamate was slowly added and the contents stirred until dissolution occurred. The solution was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate solution 150 mg/ml (chilled on ice) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Triethyl citrate | | 5 |
| Ascorbic acid | | 0.5 |
| Ultrapure water | **to** | 1 ml |
| | | |
| Solution pH | | 6.5 |
| Onset of gelation (35°C) | | 0-5 minutes |
| Viscosity of solution | | 10.2 mPa·s (cP) |

Reference Example 2, containing the same chitosan concentration as the previous reference example but with an increased ascorbic acid concentration, again exhibited a rapid onset of gelation at 35 °C using the technique described previously and the viscosity of the solution was further reduced.

### Reference Example 3: Self-gelling chitosan solution containing chitosan glutamate 18.8 mg/ml, β-glycerophosphate disodium 14.1 mg/ml, triethyl citrate 5 mg/ml and ascorbic acid 1 mg/ml

### Part A Chitosan solution

57.5 mg triethyl citrate was weighed into a glass beaker and approximately 5 ml ultrapure water added. 2.3 ml ascorbic acid solution 5 mg/ml was added to the beaker with stirring. 217 mg chitosan glutamate was slowly added and the contents stirred until dissolution occurred. The solution was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate solution 150 mg/ml (chilled on ice) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Triethyl citrate | | 5 |
| Ascorbic acid | | 1 |
| Ultrapure water | **to** | 1 ml |
| | | |
| Solution pH | | 6.4 |
| Onset of gelation (35°C) | | 0-5 minutes |
| Viscosity of solution | | 0 mPa·s (cP) |

Reference Example 3, containing chitosan glutamate 18.8 mg/ml and a further increased ascorbic acid concentration, exhibited a rapid onset of gelation at 35 °C using the technique described previously and the viscosity of the solution following manufacture was again further reduced.

### Reference Example 4: Self-gelling chitosan solution containing chitosan glutamate 14.1 mg/ml, β-glycerophosphate disodium 10.6 mg/ml, triethyl citrate 3.75 mg/ml and ascorbic acid 0.07 mg/ml

### Part A Chitosan solution

86.25 mg triethyl citrate was weighed into a glass beaker and approximately 15 ml ultrapure water added. 0.345 ml ascorbic acid solution 5 mg/ml was added to the beaker with stirring. 325.5 mg chitosan glutamate was slowly added and the contents stirred until dissolution occurred. The solution was transferred to a 20 ml volumetric flask, the washings transferred and the contents made up to volume with ultrapure water and mixed.

### Part B Self-gelling chitosan solution

13.8 ml of cold chitosan solution Part A (chilled on ice) was dispensed into a glass beaker using a Gilson Microman pipette and 1.125 ml of cold β-glycerophosphate solution 150 mg/ml (chilled on ice) was slowly added (dropwise) with vigorous stirring. 1.005 ml ultrapure water was added and stirring continued for a further 5 minutes. The onset of gelation and the solution viscosity were determined as detailed previously.

| Composition | | mg/ml |
|---|---|---|
| Chitosan glutamate | | 14.1 |
| β-glycerophosphate disodium | | 10.6 |
| Triethyl citrate | | 3.75 |
| Ascorbic acid | | 0.07 |
| Ultrapure water | **to** | 1 ml |
| | | |
| Solution pH | | 6-5 |
| Onset of gelation (35 °C) | | 10-15 minutes |
| Viscosity of solution | | 70.5 mPa·s (cP) |
| | | |
| Viscosity of control solution | | 135.9 mPa·s (cP) |
| *(14.1 mg*/*ml chitosan glutamate)* | | |

Reference Example 4, containing a reduced chitosan glutamate concentration of 14.4 mg/ml (and correspondingly reduced β-glycerophosphate and triethyl citrate concentrations), exhibited a satisfactory onset of gelation at 35°C using the technique described previously and a significantly lower viscosity than a control solution of the same polymer concentration.

### Reference Example 5: Self-gelling chitosan solution containing chitosan glutamate 14.1 mg/ml, β-glycerophospbate disodium 10.6 mg/ml, triethyl citrate 3.75 mg/ml and ascorbic acrid 0.19 mg/ml

### Part A Chitosan solution

86.25 mg triethyl citrate was weighed into a glass beaker and approximately 15 ml ultrapure water added. 0.863 ml ascorbic acid solution 5 mg/ml was added to the beaker with stirring. 325.5 mg chitosan glutamate was slowly added and the contents stirred until dissolution occurred. The solution was transferred to a 20 ml volumetric flask, the washings transferred and the contents made up to volume with ultrapure water and mixed.

### Part B Self-gelling chitosan solution

13.8 ml, of cold chitosan solution Part A (chilled on ice) was dispensed into a glass beaker using a Gilson Microman pipette and 1.125 ml of cold β-glycerophosphate solution 150 mg/ml (chilled on ice) was slowly added (dropwise) with vigorous stirring. 1.005 ml ultrapure water was added and stirring continued for a further 5 minutes. The onset of gelation and the solution viscosity were determined as detailed previously.

| Composition | | mg/ml |
|---|---|---|
| Chitosan glutamate | | 14.1 |
| β-glycerophosphate disodium | | 10.6 |
| Triethyl citrate | | 3.75 |
| Ascorbic acid | | 0.19 |
| Ultrapure water | **to** | 1 ml |
| | | |
| Solution pH | | 6.5 |
| Onset of gelation (35°C) | | 10-15 minutes |
| Viscosity of solution | | 32.7 mPa·s (cP) |

Reference Example 5, containing a reduced chitosan glutamate concentration of 14.4 mg/ml (and correspondingly reduced β-glycerophosphate and triethyl citrate concentrations) and a slightly higher ascorbic acid concentration than reference example 4, exhibited a satisfactory onset of gelation at 35 °C using the technique described previously and the viscosity of the solution was further reduced.

### Reference Example 6: Self-gelling chitosan solution containing chitosan glutamate 14.1 mg/ml, β-glycerophosphate disodium 10.6 mg/ml, triethyl citrate 3.75 mg/ml and ascorbic acid 0.37 mg/ml

### Part A Chitosan solution

86.25 mg triethyl citrate was weighed into a glass beaker and approximately 15 ml ultrapure water added. 1.725 ml ascorbic acid solution 5 mg/nnl was added to the beaker with stirring. 325.5 mg chitosan glutamate was slowly added and the contents stirred until dissolution occurred. The solution was transferred to a 20 ml volumetric flask, the washings transferred and the contents made up to volume with ultrapure water and mixed.

### Part B Self-gelling chitosan solution

13.8 ml of cold chitosan solution Part A (chilled on ice) was dispensed into a glass beaker using a Gilson Microman pipette and 1.125 ml of cold β-glycerophosphate solution 150 mg/ml (chilled on ice) was slowly added (dropwise) with vigorous stirring. 1.005 ml ultrapure water was added and stirring continued for a further 5 minutes. The onset of gelation and the solution viscosity were determined as detailed previously.

| Composition | | mg/ml |
|---|---|---|
| Chitosan glutamate | | 14.1 |
| β-glycerophosphate disodium | | 10.6 |
| Triethyl citrate | | 3.75 |
| Ascorbic acid | | 0.37 |
| Ultrapure water | **to** | 1 ml |
| | | |
| Solution pH | | 6.5 |
| Onset of gelation (35°C) | | 10-15 minutes |
| Viscosity of solution | | 1 0.2 mPa·s (cP) |

Reference Example 6, containing a reduced chitosan glutamate concentration of 14.4 mg/ml (and correspondingly reduced β-glycerophosphate and triethyl citrate concentrations) and a slightly higher ascorbic acid concentration than Reference Example 5, exhibited a satisfactory onset of gelation at 35°C using the technique described previously and the viscosity of the solution was again further reduced.

### Reference Example 7: Self-gelling chitosan solution containing chitosan glutamate 9.4 mg/ml, β-glycerophosphate disodium 7.1 mg/ml and triethyl citrate 5 mg/ml

### Part A Chitosan solution

114 mg triethyl citrate was weighed into a glass beaker and approximately 16 ml ultrapure water added. 217 mg chitosan glutamate was slowly added and the contents stirred until dissolution occurred. The solution was transferred to a 20 ml volumetric flask, the washings transferred and the contents made up to volume with ultrapure water and mixed.

### Part B Self-gelling chitosan solution

9.2 ml of cold chitosan solution Part A (chilled on ice) was dispensed into a glass beaker using a Gilson Microman pipette and 0.133 ml of cold β-glycerophosphate solution 562.5 mg/ml (chilled on ice) was slowly added (dropwise) with vigorous stirring. 1.285 ml ultrapure water was added and stirring continued for a further 5 minutes. The onset of gelation and the solution viscosity were determined as detailed previously.

| Composition | | mg/ml |
|---|---|---|
| Chitosan glutamate | | 9.4 |
| β-glycerophosphate disodium | | 7.1 |
| Triethyl citrate | | 5 |
| Ultrapure water | **to** | 1 ml |
| | | |
| Solution pH | | 6.4 |
| Onset of gelation (35°C) | | 10-15 minutes |
| Viscosity of solution | | 25.5 mPa·s (cP) |
| Viscosity of control solution | | 58.2 mPa.s (cP) |
| *(9.4 mg*/*ml chitosan glutamate)* | | |

Reference Example 7 illustrates the effect of reducing the chitosan glutamate and β-glycerophosphate concentrations to 9.4 mg/ml to 7.1 mg/ml respectively. A satisfactory onset of gelation was noted at 35 °C using the technique described previously and the formulation exhibited a lower viscosity than a control solution of similar polymer concentration.

### Reference Example 8: Self gelling chitosan solution containing chitosan glutamate 18.8 mg/ml, β-glycerophosphate disodium 14.1 mg/ml, triethyl citrate 5 mg/ml and fumaric acid 1 mg/ml

### Part A Chitosan solution

57.5 mg triethyl citrate was weighed into a glass beaker and approximately 7.5 ml ultrapure water added. 11.5 mg of fumaric acid (Sigma Aldrich, UK) was added to the beaker with stirring. 217 mg chitosan glutamate was slowly added and the contents stirred until dissolution occurred. The solution, was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate solution 150 mg/ml (chilled on ice) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Triethyl citrate | | 5 |
| Fumaric acid | | 1 |
| Ultrapure water | **to** | 1 ml |
| | | |
| Solution pH | | 6.3 |
| Onset of gelation (35 °C) | | 5-10 minutes |
| Viscosity of solution | | 95 mPa·s (cP) |

Reference Example 8 illustrates the effectiveness of fumaric acid in reducing solution viscosity for' a product containing chitosan glutamate 18.8 mg/ml. Compared with reference example 3 (of similar composition but containing ascorbic rather than fumaric acid), reference example 8 exhibited a higher solution viscosity at 25°C, which is however still significantly lower than a control solution of similar polymer concentration.

### Example 9: Self-gelling chitosan solution containing salmon calcitonin (s-CT) 0.3 mg/ml, chitosan glutamate 18.8 mg/ml, β-glycerophosphate disodium 14.1 mg/ml, triethyl citrate 5 mg/ml and ascorbic acid 1 mg/ml

### Part A Chitosan-salmon calcitonin solution

115 mg triethyl citrate was weighed into a glass beaker and approximately 5 ml ultrapure water added. 7 mg of salmon calcitonin (Polypeptide Laboratories Inc., Torrance CA, USA) was weighed into a second small beaker (silanised) and approximately 10 ml ultrapure water added. The triethyl citrate solution was transferred to the beaker containing the salmon calcitonin solution and the contents stirred. 23 mg ascorbic acid was added to the beaker with stirring. 434 mg chitosan glutamate was slowly added and the contents stirred until dissolution occurred. The solution was transferred to a 20 ml volumetric flask (silanised), the washings transferred and the contents made up to volume with ultrapure water and mixed.

### Part B Self-gelling chitosan solution containing salmon calcitonin

9.2 ml of cold chitosan-salmon calcitonin solution Part A (chilled on ice) was dispensed into a glass beaker (silanised) using a Gilson Microman pipette and 1 ml of cold β-glycerophosphate solution 150 mg/ml (chilled on ice) was slowly added (dropwise) with vigorous stirring. 0.418 ml ultrapure water was added and stirring continued for a further 5 minutes. The onset of gelation was determined as detailed previously.

| Composition | | mg/ml |
|---|---|---|
| Salmon calcitonin | | 0.3 |
| Chitosan glutamate | | 18.8 |
| β-glycerophosphate disodium | | 14.1 |
| Triethyl citrate | | 5 |
| Ascorbic acid | | 1 |
| Ultrapure water | **to** | 1 ml |
| | | |
| Solution pH | | 6.4 |
| Onset of gelation (35 °C) | | 5-10 minutes |
| Viscosity of solution | | 3.1 mPa.s (cP) |

Example 9 illustrates the effect of the incorporation of salmon calcitonin into self-gelling chitosan solution containing chitosan glutamate 18.8 mg/ml. Compared with reference example 3, of similar composition but in the absence of drug, example 9 exhibited a slightly slower onset of gelation using the technique described previously. No significant difference in viscosity was noted.

### Example 10: Self-gelling chitosan solution containing morphine 20 mg/ml (as the methanesulphonate), chitosan glutamate 18.8 mg/ml, β-glycerophosphate disodium 14.1 mg/ml, triethyl citrate 5 mg/ml and ascorbic acid 1 mg/ml

### Part A Chitosan-morphine solution

115 mg triethyl citrate was weighed into a glass beaker and approximately 17 ml ultrapure water and 0.761 ml 2M methanesulphonic acid (Sigma Aldrich, UK) were added with stirring. 462 mg of morphine monohydrate (Macfarlan Smith Ltd, UK), 23 mg of ascorbic acid and 434 mg chitosan glutamate were added to the beaker and the contents stirred until dissolution occurred. The solution was transferred to a 20 ml volumetric flask, the washings transferred and the contents made up to volume with ultrapure water and mixed.

### Part B Self-gelling chitosan solution containing morphine

92 ml of cold chitosan-morphine solution Part A (chilled on ice) was dispensed into a glass beaker using a Gilson Microman pipette and 1 ml of cold β-glycerophosphate solution 150 mg/ml (chilled on ice) was slowly added (dropwise) with vigorous stirring. 0.418 ml ultrapure water was added and stirring continued for a further 5 minutes. The onset of gelation was determined as detailed previously.

| Composition | | mg/ml |
|---|---|---|
| Morphine | | 20 |
| Chitosan glutamate | | 18.8 |
| β-glycerophosphate disodium | | 14.1 |
| Triethyl citrate | | 5 |
| Ascorbic acid | | 1 |
| Ultrapure water | **to** | 1 ml |
| | | |
| Solution pH | | 6.3 |
| Onset of gelation (3 5 °C) | | 5-10 minutes |
| Viscosity of solution | | 99.1 mPa.s (cP) |

Example 10 illustrates the effect of the incorporation of morphine 20 mg/ml (as the methanesulphonate) into self-gelling chitosan solution containing chitosan glutamate 18.8 mg/ml. Compared with reference example 3, of similar composition but in the absence of drug, example 10 exhibited a slightly slower onset of gelation using the technique described previously, although significantly, a higher solution viscosity.

### Example 11: Self gelling chitosan solution containing hydromorphone hydrochloride 4 mg/ml, chitosan glutamate 18.8 mg/ml, β-glycerophosphate disodium 14.1 mg/ml, triethyl citrate 5 mg/ml and ascorbic acid 1 mg/ml

### Part A Chitosan-hydromorphone solution

115 mg triethyl citrate was weighed into a glass beaker and approximately 15 ml ultrapure water was added with stirring. 23 mg of ascorbic acid was added to the beaker with stirring, followed by 92 mg hydromorphone hydrochloride (Macfarlan Smith Ltd, UK). 434 mg chitosan glutamate was slowly added and the contents stirred until dissolution occurred. The solution was transferred to a 20 ml volumetric flask, the washings transferred and the contents made up to volume with ultrapure water and mixed.

### Part B Self-gelling chitosan solution containing hydromorphone

9.2 ml of cold chitosan-hydromorphone solution Part A (chilled on ice) was dispensed into a glass beaker using a Gilson Microman pipette and 1 ml of cold β-glycerophosphate solution 150 mg/ml (chilled on ice) was slowly added (dropwise) with vigorous stirring. 0.418 ml ultrapure water was added and stirring continued for a further 5 minutes. The onset of gelation was determined as detailed previously.

| Composition | | mg/ml |
|---|---|---|
| Hydromorphone hydrochloride | | 4 |
| Chitosan glutamate | | 18.8 |
| β-glycerophosphate disodium | | 14.1 |
| Triethyl citrate | | 5 |
| Ascorbic acid | | 1 |
| Ultrapure water | **to** | 1 ml |
| Solution pH | | 6.3 |
| Onset of gelation (3 5 °C) | | 5-10 minutes |
| Viscosity of solution | | 11.2 mPa.s (cP) |

Example 11 illustrates the effect of the incorporation of hydromorphone hydrochloride 4 mg/ml into self-gelling chitosan solution containing chitosan glutamate 18.8 mg/ml. Compared with reference example 3, of similar composition but in the absence of drug, example 11 exhibited a slightly slower onset of gelation using the technique described previously. No significant difference in viscosity was noted.

### Example 12: Self-gelling chitosan solution containing apomorphine 5 mg/ml (as the hydrochloride), chitosan glutamate 18.8 mg/ml, β-glycerophosphate disodium 14.1 mg/ml, triethyl citrate 5 mg/ml and ascorbic acid 1 mg/ml

### Part A Chitosan-apomorphine solution

115 mg triethyl citrate was weighed into a glass beaker and approximately 15 ml ultrapure water added. 23.1 mg ascorbic acid and 118.4 mg apomorphine hydrochloride (Macfarlan Smith Ltd, UK) were added to the beaker with stirring. 434 mg chitosan glutamate was slowly added and the contents stirred until dissolution occurred. The solution was transferred to a 20 ml volumetric flask, the washings transferred and the contents made up to volume with ultrapure water and mixed.

### Part B Self-gelling chitosan solution containing apomorphine

9.2 ml of cold chitosan-apomorphine solution Part A (chilled on ice) was dispensed into a glass beaker using a Gilson Microman pipette and 1 ml of cold β-glycerophosphate solution 150 mg/ml (chilled on ice) was slowly added (dropwise) with vigorous stirring. 0.418 ml ultrapure water was added and stirring continued for a further 5 minutes. The onset of gelation and the solution viscosity were determined as detailed previously.

| Composition | | mg/ml |
|---|---|---|
| Apomorphine hydrochloride | | 5 |
| Chitosan glutamate | | 18.8 |
| β-glycerophosphate disodium | | 14.1 |
| Triethyl citrate | | 5 |
| Ascorbic acid | | 1 |
| Ultrapure water | **to** | 1 ml |
| | | |
| Solution pH | | 6.4 |
| Onset of gelation (35 °C) | | 5-10 minutes |
| Onset of gelation (3 7 °C) | | 0-5 minutes |
| Viscosity of solution | | 36.8 mPa.s (cP) |

Example 12 illustrates the effect of the incorporation of apomorphine hydrochloride 5 mg/ml into self-gelling chitosan solution containing chitosan glutamate 18.8 mg/ml. Compared with reference example 3, of similar composition but in the absence of drug, example 10 exhibited a slightly slower onset of gelation at 35°C using the technique described previously and a slightly higher viscosity.

### Example 13: Self-gelling chitosan solution containing apomorphine 5 mg/ml (as the hydrochloride), chitosan glutamate 18.8 mg/ml, β-glycerophosphate disodium 14.1 mg/ml, triethyl citrate 5 mg/ml, ascorbic acid 1 mg/ml and disodium edetate 0.05 mg/ml

### Part A Chitosan-apomorphine solution

115 mg triethyl citrate was weighed into a glass beaker and approximately 15 ml ultrapure water added. 1 ml of disodium edetate solution 1 mg/ml, 23 mg ascorbic acid and 118.4 mg apomorphine hydrochloride were added to the beaker with stirring. 434 mg chitosan glutamate was slowly added and the contents stirred until dissolution occurred. The solution was transferred to a 20 ml volumetric flask, the washings transferred and the contents made up to volume with ultrapure water and mixed.

### Part B Self-selling chitosan solution containing apomorphine

9.2 ml of cold chitosan-apomorphine solution Part A (chilled on ice) was dispensed into a glass beaker using a Gilson Microman pipette and 1 ml of cold β-glycerophosphate solution 150 mg/ml (chilled on ice) was slowly added (dropwise) with vigorous stirring. 0.418 ml ultrapure water was added and stirring continued for a further 5 minutes. The onset of gelation was determined as detailed previously.

| Composition | | mg/ml |
|---|---|---|
| Apomorphine hydrochloride | | 5 |
| Chitosan glutamate | | 18.8 |
| β-glycerophosphate disodium | | 14.1 |
| Triethyl citrate | | 5 |
| Ascorbic acid | | 1 |
| Disodium edetate | | 0.05 |
| Ultrapure water | **to** | 1 ml |
| | | |
| Solution pH | | 6.3 |
| Onset of gelation (35°C) | | 5-10 minutes |

Example 13 illustrates the effect of the incorporation of disodium edetate 0.05 mg/ml into self-gelling chitosan solution containing apomorphine hydrochloride 5 mg/ml. Compared with Example 12, of similar composition but in the absence of the antioxidant synergist, no significant difference in the product was noted.

### Example 14: Self-gelling chitosan solution containing chitosan glutamate 18.8 mg/ml, β-glycerophosphate disodium 14.1 mg/ml and polysorbate 80 10 µl/ml

### Part A Chitosan solution

434 mg chitosan glutamate was slowly added with stirring to a beaker containing approximately 18 ml ultrapure water. The contents were stirred until dissolution occurred. The solution was transferred to a 20 ml volumetric flask, the washings transferred and the contents made up to volume with ultrapure water and mixed.

### Part B Self-gelling chitosan solution

9.2 ml of cold chitosan solution Part A (chilled on ice) was dispensed into a glass beaker using a Gilson Microman pipette and 0.266 ml of cold β-glycerophosphate solution 562.5 mg/ml (chilled on ice) was slowly added (dropwise) with vigorous stirring. 0.106 ml of polysorbate 80 (Sigma Aldrich, UK) and 1.046 ml ultrapure water were added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Polysorbate 80 | | 10 µl |
| Ultrapure water | **to** | 1 ml |
| | | |
| Solution pH | | 6.2 |
| Onset of gelation (37 °C) | | 5-10 minutes |
| Viscosity of solution | | 114.4 mPa.s (cP) |

Example 14 illustrates the effectiveness of polysorbate 80 in facilitating a rapid onset of gelation for a product containing chitosan glutamate 18.8 mg/ml. An onset of gelation of 5-10 minutes at 37 °C and a solution viscosity of 114 mPa·s (cP) was noted, confirming the suitability of polysorbate 80 mPa·s as an alternative to triethyl citrate in self-gelling chitosan solution.

### Example 15: Self-gelling chitosan solution containing chitosan glutamate 18.8 mg/ml, β-glycerophosphate disodium 14.1 mg/ml, triethyl citrate 5 mg/ml and malic acid 1 mg/ml

### Part A Chitosan solution

57.5 mg triethyl citrate was weighed into a glass beaker and approximately 7.5 ml ultrapure water added. 11.5 mg of malic acid (Sigma Aldrich, UK) was added to the beaker with stirring. 217 mg chitosan glutamate was slowly added and the contents stirred until dissolution occurred. The solution was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate solution 150 mg/ml (chilled on ice) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Triethyl citrate | | 5 |
| Malic acid | | 1 |
| Ultrapure water | **to** | 1 ml |
| | | |
| Solution pH | | 6.1 |
| Onset of gelation (35°C) | | 5-10 minutes |
| Viscosity of solution | | 113.4 mPa·s (cP) |

Example 15 illustrates the effectiveness of malic acid in reducing solution viscosity for product containing chitosan glutamate 18.8 mg/ml. Compared with reference example 3 (of similar composition but containing ascorbic rather than malic acid), Example 15 exhibited a higher solution viscosity at 25°C, which is however still significantly lower than a control solution of similar polymer concentration.

### Example 16: Self-gelling chitosan solution containing chitosan glutamate 18.8 mg/ml, β0-glycerophosphate disodium 14.1 mg/ml, triethyl citrate 5 mg/ml and tartaric acid 1 mg/ml

### Part A Chitosan solution

57.5 mg triethyl citrate was weighed into a glass beaker and approximately 7.5 ml ultrapure water added. 11.5 mg of tartaric acid (Sigma Aldrich, UK) was added to the beaker with stirring. 217 mg chitosan glutamate was slowly added and the contents stirred until dissolution occurred. The solution was transferred to a 10 ml volumetric flask, the washings transferred and the contents made up to volume with ultrapure water and mixed.

### Part B Self-gelling chitosan solution

4.6 ml of cold chitosan solution Part A (chilled on ice) was dispensed into a glass beaker using a Gilson Microman pipette and 0.5 ml of cold β-glycerophosphate solution 150 mg/ml (chilled on ice) was slowly added (dropwise) with vigorous stirring. 0.209 ml ultrapure water was added and stirring continued for a further 5 minutes. The onset of gelation and the solution viscosity were determined as detailed previously.

| Composition | | mg/ml |
|---|---|---|
| Chitosan glutamate | | 18.8 |
| β-glycerophosphate disodium | | 14.1 |
| Triethyl citrate | | 5 |
| Tartaric acid | | 1 |
| Ultrapure water | to | 1 ml |
| | | |
| Solution pH | | 6.3 |
| Onset of gelation (35 °C) | | 10-15 minutes |
| Viscosity of solution | | 83.8 mPa.s (cP) |

Example 16 illustrates the effectiveness of tartaric acid in reducing solution viscosity for product containing chitosan glutamate 18.8 mg/ml. Compared with Reference Example 3 (of similar composition but containing ascorbic rather than tartaric acid), Example 16 exhibited a higher solution viscosity at 25 °C, which is however still significantly lower than a control solution of similar polymer concentration.

### Example 17: Self-gelling chitosan solution containing salmon calcitonin 0.368 mg/ml (2000 IU/ml), chitosan glutamate 18.8 mg/ml, β-glycerophosphate disodium 14.1 mg/ml, triethyl citrate 5 mg/ml and ascorbic acid 0.25 mg/ml

### Part A Chitosan-salmon calcitonin solution

287.5 mg triethyl citrate was weighed into a silanised 100 nil glass beaker and approximately 38 ml ultrapure water added. 2.875 ml of ascorbic acid solution 5 mg/ml (prepared by dissolving 100 mg of ascorbic acid in ultrapure water in a 20 ml volumetric flask and making up to volume) was added to the triethyl citrate solution with stirring. 4.25 ml of salmon calcitonin solution 5 mg/ml {prepared by dissolving 100 mg of salmon calcitonin (Polypeptide Laboratories Inc., Torrance CA, USA) in ultrapure water in a silanised 20 ml volumetric flask and making up to volume} was added to the beaker with stirring. 1085 mg chitosan glutamate was slowly added and the contents stirred until the chitosan had dissolved. The solution was transferred to a 50 ml volumetric flask (silanised), the washings transferred and the contents made up to volume with ultrapure water and mixed.

### Part B Self-gelling chitosan solution containing salmon calcitonin

43.323 ml of cold chitosan-salmon calcitonin solution Part A (chilled on ice) was dispensed into a glass beaker (silanised) using a Gilson pipette and 4.71 ml of cold β-glycerophosphate solution 150 mg/ml (chilled on ice) was slowly added (dropwise) with vigorous stirring. 1.968 ml ultrapure water was added and stirring continued for a further 5 minutes. The onset of gelation was determined as detailed previously.

| Composition | | mg/ml |
|---|---|---|
| Salmon calcitonin | | 0.368 (equivalent to 2000 IU/ml) |
| Chitosan glutamate | | 18.8 |
| β-glycerophosphate disodium | | 14.1 |
| Triethyl citrate | | 5 |
| Ascorbic acid | | 0.25 |
| Ultrapure water | **to** | 1 ml |
| | | |
| Solution pH | | 6.3 |
| Onset of gelation (35 °C) | | 0-5 minutes |
| Viscosity of solution | | 39.6 mPa.s (cP) |

Example 17 illustrates the effect of the incorporation of salmon calcitonin 2000 IU/ml into self-gelling chitosan solution containing chitosan glutamate 18.8 mg/ml. Compared with Example 9, of similar composition but containing 1 mg/ml ascorbic acid, Example 17 exhibited a slightly higher solution viscosity and a slightly faster onset of gelation using the technique described previously. No significant difference in solution pH was noted.

### Example 18: Intranasal absorption of self-gelling chitosan solution containing salmon calcitonin 2000 IU/ml in sheep

Self-gelling chitosan solution containing 2000 IU/ml s-CT and 18.8 mg/ml chitosan glutamate described in Example 17, was dosed to sheep in a study designed to determine the influence of self-gelling chitosan solution on its effectiveness as an intranasal absorption enhancer. The absorption of salmon calcitonin in sheep was evaluated from self-gelling chitosan solution containing 18.8 mg/ml chitosan and from a nasal control solution. The formulations dosed were as follows:
Formulation 1 (F1) Salmon calcitonin solution (control) containing 2000 IU/ml s-CT
Formulation 2 (F2) Self-gelling chitosan solution containing 2000 IU/ml s-CT and 18.8 mg/ml chitosan glutamate

The method of preparation of the self-gelling chitosan solution is as described in Example 17. The control solution was prepared by dispensing approximately 35 ml ultrapure water into a small glass beaker and adding 425 mg sodium chloride (Sigma Aldrich, UK) with stirring. When the sodium chloride had dissolved, the solution was transferred to a silanised 50 ml volumetric flask and 2.5 ml benzethonium chloride solution 3 mg/ml (prepared by dissolving 60 mg benzethonium chloride (Sigma Aldrich, UK) in 20 ml ultrapure water) was added with stirring, followed by 3.683 ml of salmon calcitonin solution 5 mg/ml. The pH of the solution was adjusted to pH 4 using 0.1M HCl, the contents made up to volume and the solution mixed thoroughly.

A nominal dose of 0.6 ml of each formulation (1200 IU) was dosed to each of six sheep. Blood samples were collected and the plasma separated. Quantitative analysis of salmon calcitonin in plasma was performed using an ELISA method. Pharmacokinetic analysis of data was performed using a WinNonlin program for PC (Scientific Consulting Inc., North Carolina, USA). A summary of the pharmacokinetic data is provided in Table 1. Mean plasma concentration-time curves are presented in Figure 1.

As a simple solution, salmon calcitonin was reasonably well absorbed from the nasal cavity of the sheep. For the self-gelling chitosan solution, a marked increase in Cₘₐₓ, AUC and relative bioavailability (Fᵣₑₗ) was noted. In addition the Tₘₐₓ was also extended confirming prolonged residence time between the formulation and the mucosal surface.

In conclusion the study demonstrated the ability of self-gelling chitosan solution to enhance the residence time between the formulation and the nasal mucosa, with the consequence of enhanced bioavailability of salmon calcitonin following the intranasal administration to sheep.

**Table 1 Summary pharmacokinetic parameters**

| **Formulation** | **PK parameters** | **Mean** | **SD** | **CV(%)** |
|---|---|---|---|---|
| F1: nasal control solution | Tₘₐₓ(min) | 10 | 6 | 60 |
| | Cₘₐₓ (pg/ml) | 60.0 | 26.0 | 43 |
| | AUCₗₐₛₜ (pg.min/ml) | 993 | 618 | 62 |
| | Dose (IU) | 1200 | 0 | 0 |
| | Fᵣₑₗ (%) | 100 | - | |
| F2: self-gelling chitosan solution | Tₘₐₓ (min) | 18 | 10 | 56 |
| | Cₘₐₓ (pg/ml) | 148.0 | 44.0 | 30 |
| | AUCₗₐₛₜ (pg.min/ml) | 6520 | 4648 | 71 |
| | Dose(IU) | 1200 | 0 | 0 |
| | Fᵣₑₗ(%) | 657 | 468 | 71 |

| | | | | |
|---|---|---|---|---|
| Fᵣₑₗ (%): bioavailability relative to nasal control solution | | | | |

## Claims

1. A nasal or ocular drug delivery composition in the form of an aqueous solution or suspension for delivery of a therapeutic agent across a nasal or ocular mucosal surface into the systemic circulation comprising:
(i) chitosan, a salt thereof or a derivative thereof that has been formed by bonding of acyl or alkyl groups with the hydroxyl groups of the chitosan or a salt of such a derivative thereof;
(ii) a polyol-phosphate or sugar-phosphate salt;
(iii) triethyl citrate as a plasticizer; and
(iv) a systemically acting therapeutic agent.

2. A composition according to claim 1 for nasal delivery.

3. A composition according to claim 1 or 2, wherein the polyol-phosphate salt is β-glycerophosphate disodium.

4. A composition according to any one of the preceding claims, wherein the chitosan, salt thereof or derivative thereof or a salt of a derivative thereof has a molecular weight of 4000 Dalton or greater.

5. A composition according to claim 4, wherein the chitosan, salt thereof or derivative thereof or salt of a derivative thereof, has a molecular weight of from 50,000 to 300,000 Dalton.

6. A composition according to any one of the preceding claims, comprising chitosan base or a nitrate, phosphate, sulphate, citrate, hydrochloride, glutamate, lactate or acetate salt of chitosan.

7. A composition according to any one of the preceding claims, wherein the chitosan has a degree of deacetylation of 40% or greater.

8. A composition according to claim 7, wherein the degree of deacetylation is from 70 to 90%.

9. A composition according to any of the preceding claims comprising from 0.25 to 3.0% w/v of chitosan, a salt or a derivative thereof or a salt of a derivative thereof expressed as chitosan base.

10. A composition according to claim 9 comprising from 0.45 to 1.5 %w/v of chitosan, a salt or a derivative thereof or a salt of a derivative thereof expressed as chitosan base.

11. A composition according to any one of the preceding claims, wherein the polyol-phosphate or sugar-phosphate salt is present in an amount of from 0.25 to 3.0 % w/v.

12. A composition according to claim 11, wherein the polyol-phosphate or sugar-phosphate salt is present in an amount of from 0.75 to 2.0 % w/v.

13. A composition according to any one of the preceding claims comprising from 0.05 to 5.0 % w/v of triethyl citrate.

14. A composition as claimed in claim 13 comprising from 0.2 to 1.0 % w/v of triethyl citrate.

15. A composition according to any of the preceding claims additionally comprising ascorbic acid.

16. A composition according to claim 15 comprising from 0.01 to 0.2 % w/v ascorbic acid.

17. A composition according to any one of the preceding claims, wherein the therapeutic agent is a polar drug, a polypeptide, a gene or a gene construct.

18. A composition according to claim 17, wherein the therapeutic agent is insulin, calcitonin, leuprolide, luteinising hormone releasing hormone, growth hormone or a growth hormone releasing factor, naratriptan, sumatriptan, zolmitriptan, rizatriptan, eletriptan, frovatriptan, alnitidan, avitriptan, almotriptan, apomorphine, sildenafil, alprostadil, diamorphine, hydromorphone, buprenorphine, fentanyl, oxycodone, codeine, morphine or morphine-6-glucuronide.

19. A drug delivery device suitable for delivery of a composition via one or more of the nasal or ocular routes or a dose cartridge for use with such a device loaded with a composition as defined in any one of the preceding claims.

20. A process for the preparation of the composition as defined in any one of claims 1 to 18, which process comprises mixing a solution comprising chitosan or a salt thereof or derivative thereof or a salt of a derivative thereof with a solution comprising a polyol-phosphate or sugar-phosphate salt.

21. The use of the combination of chitosan or a salt thereof or derivative thereof that has been formed by bonding of acyl or alkyl groups with the hydroxyl groups of the chitosan or a salt of such a derivative thereof, a polyol-phosphate or sugar-phosphate salt and triethyl citrate as a plasticizer in the manufacture of a medicament for nasal or ocular delivery of a systemically acting therapeutic agent.

## Patentansprüche

1. Nasale oder okulare Arzeimittelabgabezusammensetzung in Form einer wässrigen Lösung oder Suspension für die Abgabe eines therapeutischen Mittels durch eine nasale oder okulare Schleimhautoberfläche in die systemische Zirkulation, umfassend:
(i) Chitosan, ein Salz davon oder ein Derivat davon, das durch Binden von Acyl- oder Alkylgruppen an die Hydroxylgruppen des Chitosans oder eines Salzes eines derartigen Derivats davon gebildet worden ist;
(ii) ein Polyolphosphat- oder Zuckerphosphatsalz;
(iii) Triethylcitrat als Weichmacher; und
(iv) ein systemisch wirkendes therapeutisches Mittel.

2. Zusammensetzung nach Anspruch 1 für die nasale Abgabe.

3. Zusammensetzung nach Anspruch 1 oder 2, wobei das Polyolphosphatsalz β-Glycerophosphat-Dinatrium ist.

4. Zusammensetzung nach einem der vorhergehenden Ansprüche, wobei das Chitosan, Salz davon oder Derivat davon oder ein Salz eines Derivats davon ein Molekulargewicht von 4000 Dalton oder mehr aufweist.

5. Zusammensetzung nach Anspruch 4, wobei das Chitosan, Salz davon oder Derivat davon oder Salz eines Derivats davon ein Molekulargewicht von 50.000 bis 300.000 Dalton aufweist.

6. Zusammensetzung nach einem der vorhergehenden Ansprüche, umfassend Chitosanbase oder ein Nitrat-, Phosphat-, Sulfat-, Citrat-, Hydrochlorid-, Glutamat-, Lactat- oder Acetatsalz von Chitosan.

7. Zusammensetzung nach einem der vorhergehenden Ansprüche, wobei das Chitosan einen Deacetylierungsgrad von 40 % oder mehr aufweist.

8. Zusammensetzung nach Anspruch 7, wobei der Deacetylierungsgrad 70 bis 90 % beträgt.

9. Zusammensetzung nach einem der vorhergehenden Ansprüche umfassend 0,26 bis 3,0 Gew.-/Vol.-% Chitosan, eines Salzes oder eines Derivats davon oder eines Salzes eines Derivats davon, als Chitosanbase ausgedrückt.

10. Zusammensetzung nach Anspruch 9 umfassend 0,45 bis 1,5 Gew.- /Vol.-% Chitosan, eines Salzes oder eines Derivats davon oder eines Salzes eines Derivats davon, als Chitosanbase ausgedrückt.

11. Zusammensetzung nach einem der vorhergehenden Ansprüche, wobei das Polyolphosphat- oder Zuckerphosphatsalz in einer Menge von 0,25 bis 3,0 Gew.-/Vol.-% vorliegt.

12. Zusammensetzung nach Anspruch 11, wobei das Polyolphosphat- oder Zuckerphosphatsalz in einer Menge von 0,75 bis 2,0 Gew.-/Vol.-% vorliegt.

13. Zusammensetzung nach einem der vorhergehenden Ansprüche, umfassend 0,05 bis 5,0 Gew.-/Vol.-% Triethylcitrat.

14. Zusammensetzung nach Anspruch 13, umfassend 0,2 bis 1,0 Gew.- /Vol.-% Triethylcitrat.

15. Zusammensetzung nach einem der vorhergehenden Ansprüche, zusätzlich Ascorbinsäure umfassend.

16. Zusammensetzung nach Anspruch 15, 0,01 bis 0,2 Gew.-Nol.-% Ascorbinsäure umfassend.

17. Zusammensetzung nach einem der vorhergehenden Ansprüche, wobei das therapeutische Mittel ein polares Arzneimittel, ein Polypeptid, ein Gen oder ein Genkonstrukt ist.

18. Zusammensetzung nach Anspruch 17, wobei das therapeutische Mittel Insulin, Calcitonin, Leuprolid, luteinisierendes Hormonfreisetzungshormon, Wachstumshormon oder ein Wachstumshormonfreisetzungsfaktor, Naratriptan, Sumatriptan, Zolmitriptan, Rizatriptan, Eletriptan, Frovatriptan, Alnitidan, Avitriptan, Almotriptan, Apomorphin, Sildenafil, Alprostadil, Diamorphin, Hydromorphon, Buprenorphin, Fentanyl, Oxycodon, Codein, Morphin oder Morphin-6-Glucuronid ist.

19. Arzneimittelabgabevorrichtung, die für die Abgabe einer Zusammensetzung über einen oder mehrere der nasalen oder okularen Wege oder eine Dosierkartusche geeignet ist, zur Verwendung mit einer derartigen Vorrichtung, die mit einer Zusammensetzung, wie in einem der vorhergehenden Ansprüche definiert, beladen ist.

20. Verfahren für die Herstellung der Zusammensetzung, wie in einem der Ansprüche 1 bis 18 definiert, welches Verfahren das Mischen einer Lösung, die Chitosan oder ein Salz davon oder ein Derivat davon oder ein Salz eines Derivats davon umfasst, mit einer Lösung umfassend ein Polyolphosphat- oder Zuckerphosphatsalz umfasst.

21. Verwendung der Kombination von Chitosan oder einem Salz davon oder Derivat davon, das durch Binden von Acyl- oder Alkylgruppen an die Hydroxylgruppen des Chitosans oder eines Salzes eines derartigen Derivats davon gebildet worden ist, einem Polyolphosphat- oder Zuckerphosphatsalz und von Triethylcitrat als Weichmacher bei der Herstellung eines Medikaments für die nasale oder okulare Abgabe eines systemisch wirkenden therapeutischen Mittels.

## Revendications

1. Composition pour la délivrance nasale ou oculaire d'une substance médicamenteuse, sous la forme d'une solution ou suspension aqueuse, pour la délivrance d'un agent thérapeutique à travers une surface muqueuse nasale ou oculaire jusque dans la circulation systémique, comprenant :
(i) du chitosane, un sel de celui-ci ou un dérivé de celui-ci qui a été formé en liant des groupes acyles ou alkyles avec les groupes hydroxyles du chitosane, ou un sel d'un tel dérivé de celui-ci ;
(ii) un sel polyol-phosphate ou sucre-phosphate ;
(iii) du citrate de triéthyle en tant que plastifiant ; et
(iv) un agent thérapeutique agissant systémiquement.

2. Composition selon la revendication 1, pour une délivrance nasale.

3. Composition selon la revendication 1 ou 2, dans laquelle le sel polyol-phosphate est le β-glycérophosphate disodique.

4. Composition selon l'une quelconque des revendications précédentes, dans laquelle le chitosane, le sel de celui-ci ou le dérivé de celui-ci ou un sel d'un dérivé de celui-ci a un poids moléculaire de 4000 daltons ou plus.

5. Composition selon la revendication 4, dans laquelle le chitosane, le sel de celui-ci ou le dérivé de celui-ci ou le sel d'un dérivé de celui-ci a un poids moléculaire de 50 000 à 300 000 daltons.

6. Composition selon l'une quelconque des revendications précédentes, comprenant du chitosane base ou un sel nitrate, phosphate, sulfate, citrate, chlorhydrate, glutamate, lactate ou acétate de chitosane.

7. Composition selon l'une quelconque des revendications précédentes, dans laquelle le chitosane a un degré de désacétylation de 40 % ou plus.

8. Composition selon la revendication 7, dans laquelle le degré de désacétylation est de 70 à 90 %.

9. Composition selon l'une quelconque des revendications précédentes, comprenant de 0,25 à 3,0 % p/v de chitosane, d'un sel ou d'un dérivé de celui-ci ou d'un sel d'un dérivé de celui-ci, exprimé en chitosane base.

10. Composition selon la revendication 9, comprenant de 0,45 à 1,5 % p/v de chitosane, d'un sel ou d'un dérivé de celui-ci ou d'un sel d'un dérivé de celui-ci, exprimé en chitosane base.

11. Composition selon l'une quelconque des revendications précédentes, dans laquelle le sel polyol-phosphate ou sucre-phosphate est présent en une quantité de 0,25 à 3,0 % p/v.

12. Composition selon la revendication 11, dans laquelle le sel polyol-phosphate ou sucre-phosphate est présent en une quantité de 0,75 à 2,0 % p/v.

13. Composition selon l'une quelconque des revendications précédentes, comprenant de 0,05 à 5,0 % p/v de citrate de triéthyle.

14. Composition selon la revendication 13, comprenant de 0,2 à 1,0 % p/v de citrate de triéthyle.

15. Composition selon l'une quelconque des revendications précédentes, comprenant de plus de l'acide ascorbique.

16. Composition selon la revendication 15, comprenant de 0,01 à 0,2 % p/v d'acide ascorbique.

17. Composition selon l'une quelconque des revendications précédentes, dans laquelle l'agent thérapeutique est une substance médicamenteuse polaire, un polypeptide, un gène ou une construction génique.

18. Composition selon la revendication 17, dans laquelle l'agent thérapeutique est l'insuline, la calcitonine, le leuprolide, l'hormone libérant de l'hormone lutéinisante, une hormone de croissance ou un facteur libérant une hormone de croissance, le naratriptan, le sumatriptan, le zolmitriptan, le rizatriptan, l'élétriptan, le frovatriptan, l'alnitidan, l'avitriptan, l'almotriptan, l'apomorphine, le sildénafil, l'alprostadil, la diamorphine, l'hydromorphone, la buprénorphine, le fentanyl, l'oxycodone, la codéine, la morphine ou le morphine-6-glucuronide.

19. Dispositif de délivrance d'une substance médicamenteuse convenant à la délivrance d'une composition par l'intermédiaire d'une ou plusieurs des voies nasales ou oculaires, ou cartouche doseuse pour l'utilisation avec un tel dispositif chargé avec une composition telle que définie dans l'une quelconque des revendications précédentes.

20. Procédé pour la préparation de la composition telle que définie dans l'une quelconque des revendications 1 à 18, lequel procédé comprend le mélange d'une solution comprenant du chitosane ou un sel de celui-ci ou un dérivé de celui-ci ou un sel d'un dérivé de celui-ci avec une solution comprenant un sel polyol-phosphate ou sucre-phosphate.

21. Utilisation de la combinaison de chitosane ou d'un sel de celui-ci ou d'un dérivé de celui-ci qui a été formé en liant des groupes acyles ou alkyles avec les groupes hydroxyles du chitosane ou d'un sel d'un tel dérivé de celui-ci, d'un sel polyol-phosphate ou sucre-phosphate et de citrate de triéthyle en tant que plastifiant, dans la fabrication d'un médicament pour la délivrance nasale ou oculaire d'un agent thérapeutique agissant systémiquement.
